# EUROPEAN PATENT SPECIFICATION

(11) **EP 3 335 694 B1**
(45) Date of publication and mention of the grant of the patent: **27.09.2023**
(21) Application number: 16835217.7
(22) Date of filing: 10.08.2016
(51) Int. Cl.: A61K 8/97, A61Q 19/02, A61Q 13/00

(54) **MELANOGENESIS INHIBITOR**
MELANOGENESEHEMMER
INHIBITEUR DE LA MÉLANOGENÈSE

(30) Priority: 10.08.2015 JP 2015158138
(43) Date of publication of application: 20.06.2018
(73) Proprietor: Takasago International Corporation, Tokyo 144-8721 (JP)
(72) Inventor: NARUKAMI Toshiaki, Hiratsuka-shi Kanagawa 254-0073 (JP); TERADA Ikuo, Hiratsuka-shi Kanagawa 254-0073 (JP)
(74) Representative: Fédit-Loriot
(86) International application number: PCT/JP2016/073624
(87) International publication number: WO 2017/026520

(56) References cited:
- EP-A1- 2 524 959
- CN-A- 102 872 166
- JP-A- H1 036 246
- DATABASE GNPD [Online] MINTEL; 5 February 2015 (2015-02-05), anonymous: "Fairness Cream for Sensitive Skin", XP055568706, retrieved from www.gnpd.com Database accession no. 2901643
- DATABASE GNPD [Online] MINTEL; 20 March 2014 (2014-03-20), anonymous: "Skin Brightening Eau de Toilette", XP055568710, retrieved from www.gnpd.com Database accession no. 2352480
- DATABASE GNPD [Online] MINTEL; 1 April 2015 (2015-04-01), anonymous: "Cleanser", XP055568718, retrieved from www.gnpd.com Database accession no. 2983935
- PENG HSIN-YI ET AL.: 'Effect of Vetiveria zizanioides Essential Oil on Melanogenesis in Melanoma Cells:Downregulation of Tyrosinase Expression and Suppression of Oxidative Stress' THE SCIENTIFIC WORLD JOURNAL vol. 2014, 19 March 2014, pages 1 - 9, XP055364197
- KIM HYUN-JIN ET AL.: 'Evaluation of Antioxidant Activity of Vetiver(Vetiveria zizanioides L.) Oil and Identification of Its Antioxidant Constituents' JOURNAL AGRICULTURAL FOOD CHEMISTRY vol. 53, 2005, pages 7691 - 7695, XP055364203
- DATABASE GNPD [Online] MINTEL; 1 April 2015 (2015-04-01), anonymous: "Cleanser", XP055569003, Database accession no. 2983935
- DATABASE GNPD [Online] MINTEL; 24 January 2014 (2014-01-24), anonymous: "Hydrating Balancing Aroma", XP055568724, Database accession no. 2300577
- DATABASE GNPD [Online] MINTEL; 24 April 2015 (2015-04-24), anonymous: "Moisturizer", XP055568700, Database accession no. 3089377

## Description

### Technical Field

The present invention relates to a melanin production inhibitor that inhibits darkening of a skin due to ultraviolet rays or the like, and a skin-whitening agent containing the melanin production inhibitor, which is excellent in skin-whitening effect and is highly safe.

### Background Art

When a skin is exposed to ultraviolet rays contained in sunlight, ultraviolet lamp, or the like, the skin loses the glow, fine texture, moisture, and the like. Especially, when dermis is damaged by ultraviolet rays, wrinkles and sagging are caused, which cause the so-called photoaging.

Active oxygen generated by exposure to ultraviolet rays and various factors released from cells of the skin because of the influence of the active oxygen enhance tyrosinase activity in melanocytes. Melanin which has a relation to the color tone of the skin is produced by oxidation of tyrosine with tyrosinase in melanocytes. It is thought that activation of tyrosinase by ultraviolet rays results in excessive production of melanin, and the melanin is transferred to epidermal cells, so that the color tone of the skin changes and darkens.

Hence, it is known that the inhibition of melanin production is effective in obtaining a skin-whitening effect. Examples of conventional active ingredients include ascorbic acid, kojic acid, arbutin, ellagic acid, 4-alkylresorcinols or derivatives thereof, and various plant extracts. In addition, besides these active ingredients, the followings are known: a high melanin production-inhibitory effect is exhibited when a compound which is an ester of menthol with a long-chain unsaturated fatty acid having 18 or more carbon atoms is blended with an active ingredient such as an agent for inhibiting a transmitter produced by keratinocytes, an antioxidant, an antiinflammatory, a polymer compound, and a polyvalent alcohol, because of a synergistic effect of each combination (see, for example, Patent Document 1); cedrol that is a component of sandalwood oil exhibits a melanin production-inhibitory effect (see, for example, Patent Document 2); and nerolidol that is a component of cabreuva oil exhibits a melanin production-inhibitory effect (see, for example, Patent Document 3).

Meanwhile, it was known that a vetiver extract exhibits a melanin production-promotion effect (see, for example, Patent Document 4).

The Narüko "Celestal White Moss skincare^{®}" brightening Eau de Toilette is said to evoke a relaxing and refreshing deep forest and to provide the complexion with whitening, comprising, as standard form, some 25 compounds including Juniperus virginiana oil (cedarwood). However, it is not clear which compound amongst these 25 compounds is used for brightening the skin, not to mention the use for inhibiting melanin production, subject of claim 1.

The cleanser "Elemis anti-ageing white brightening even tone^{®}" is said, inter alia, to block the synthesis of tyrosinase responsible for melanin production and to prevent future darkening, comprising, as a standard form, some 47 compounds including Myrocarpus Fastigiatus oil (cabreuva). Likewise, it is not clear which compound amongst these 47 compounds is used for blocking the synthesis of tyrosinase and for preventing future darkening.

JP H10 36246 discloses a melanin production inhibitor and skin external preparation containing cedrol as the active component.

### Citation List

### Patent Document

Patent Document 1: JP-A-2007-161591
Patent Document 2: JP-A-H10-36246
Patent Document 3: JP-A-H06-72855
Patent Document 4: JP-A-2011-157317

### Summary of Invention

### Technical Problem

In the light of the above, though a variety of components which inhibit the production of melanin are known, the harmfulness of the ultraviolet rays has been widely recognized in recent years, and it is demanded to provide a novel melanin production inhibitor capable of safely inhibiting darkening due to ultraviolet rays or the like.

### Solution to Problem

In order to solve the foregoing problem, the present inventors made extensive and intensive investigations and widely performed screening with a variety of substances with respect to the melanin production-inhibitory effect. As a result, they have found that cabreuva oil has a melanin production-inhibitory effect, thereby leading to accomplishment of the present invention.

That is, the present invention relates to the use of claim 1.

### Advantageous Effects of Invention

In accordance with the present invention, the melanin production can be effectively inhibited by the use of claim 1. In addition, a skin external agent containing this melanin production inhibitor, which is stable, highly safe, and excellent in skin-whitening effect, is provided.

### Description of Embodiments

The present invention is hereunder described in detail.

It is to be noted that in the present specification, the term "mass" is synonymous with "weight".

The plant for obtaining the melanin production inhibitor is cabreuva (Myrocarpus fastigiatus) (Leguminosae). In the present invention, cabreuva oil that is essential oil thereof is used externally for inhibiting melanin production in skin.

As for this essential oil, plant essential oil obtained through preparation from the above-described plant can be used. In addition, this essential oil is marketed, and commercially available products may also be used.

The melanin production inhibitor includes, as an active ingredient, cabreuva oil as described above.

The content of cabreuva oil, which is the active ingredient, is preferably 10% by mass or more, more preferably 30% by mass or more, still more preferably 60% by mass or more, and especially preferably 80% by mass or more, in the melanin production inhibitor. It is the most preferred that the melanin production inhibitor consists of the above-described plant oil.

The melanin production inhibitor can be used as a mixture with other conventional melanin production inhibitor (for example, pantetheine-s-sulfonic acid, isoferulic acid, ascorbic acid and derivatives thereof, hydroquinone and derivatives thereof, arbutin, kojic acid, linoleic acid (ester), ellagic acid, glycyrrhizic acid, lactic acid (ester), 4-alkylresorcinols, licorice extract, placenta extract, or the like).

Moreover, by being mixed with various bases, additives or the like, the melanin production inhibitor can be used as, for example, fragrance compositions, skin-whitening agents, skin-whitening cosmetics, fragrances or cosmetics, skin external agents, skin-whitening emulsion cosmetics, skin-whitening creams, skin-whitening lotions, skin-whitening oil-based cosmetics, skin-whitening packs, skin-whitening foundations or the like. In addition, it is possible to provide a synergistic effect by mixing with another skin-whitening agent or the like. In the above-described fragrance composition or the like, the blended amount of the melanin production inhibitor can be suitably set by those skilled in the art depending on a desired melanin production-inhibitory action or the like, and the melanin production inhibitor is blended in an amount of preferably 0.00001 to 10% by mass, and more preferably 0.0001 to 1% by mass, based on the total amount of a final product.

The melanin production inhibitor, and the products such as fragrances or cosmetics, skin external agents, etc., can be produced according to commonly adopted formulation production methods.

In the products such as fragrances or cosmetics, skin external agents, etc., in addition to the above-described one or more melanin production inhibitors as an essential constituent component, other components which are generally used for fragrances or cosmetics and skin external agents, such as cosmetics or toiletries, pharmaceuticals or the like, can be suitably blended, as the need arises, and examples of the other components include powder components, liquid fatty oils, solid fats, waxes, hydrocarbon oils, higher fatty acids, higher alcohols, synthetic ester oils, silicones, anionic surfactants, cationic surfactants, amphoteric surfactants, nonionic surfactants, humectants, water-soluble polymer compounds, thickeners, coating agents, ultraviolet absorbers, ultraviolet screening agents, preservatives, metal ion-sequestering agents, lower alcohols, polyhydric alcohols, saccharides, amino acid derivatives, organic amines, synthetic resin emulsions, pH adjusters, skin nutrients, vitamins, antioxidants, antioxidant aids, fragrances, water, and the like.

Moreover, other active medical ingredients and bioactive components, such as vitamins, skin activators, blood circulation promoters, indigenous bacteria-controlling agents, active oxygen scavengers, antiinflammatories, other skin-whitening agents, microbicides, etc., can be suitably blended, as the need arises.

Although specific examples of the components which can be blended are enumerated below, the fragrances or cosmetics, the skin external agents, and the like can be produced in the usual way upon blending the above-described essential component(s) with one or more of these optional components.

Examples of the powder components include talc, kaolin, mica, magnesium carbonate, calcium carbonate, aluminum silicate, barium silicate, calcium silicate, magnesium silicate, strontium silicate, metal tungstate, magnesium, silica, zeolite, barium sulfate, calcined calcium sulfate (burnt gypsum), calcium phosphate, hydroxyapatite, ceramic powder, metal soap (e.g., zinc myristate, calcium palmitate, and aluminum stearate); organic powders such as polyamide resin powder (nylon powder), polyethylene powder, polymethyl methacrylate powder, polystyrene powder, styrene-acrylic acid copolymer resin powder, cellulose powder, etc.; inorganic white pigments such as titanium dioxide, zinc oxide, etc.; inorganic reddish pigments such as iron oxide (Bengal red), iron titanate, etc.; inorganic violetish pigments such as carbon black, mango violet, cobalt violet, etc.; inorganic greenish pigments such as cobalt titanate, etc.; inorganic bluish pigments such as ultramarine, Prussian blue, etc.; pearl pigments such as titanium oxide-coated mica, titanium oxide-coated bismuth oxychloride, titanium oxide-coated talc, colored titanium oxide-coated mica, bismuth oxychloride, fish scale foil, etc.; metal powder pigments such as aluminum powder, copper powder, etc.; organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404, etc.; organic pigments such as zirconium, barium, or aluminum lake, etc. (e.g., Red. No. 3, Red. No. 104, Red. No. 106, Red. No. 227, Red. No. 230, Red. No. 401, Red. No. 505, Orange No. 205, Yellow No. 4, Yellow No. 5, Yellow No. 202, Yellow No. 203, Green No. 3, Blue No. 1, etc.); natural colorants such as chlorophyll, β-carotene, etc.; and the like. However, this powder component is not limited to the above-described components so long as it is a powder capable of being applied for general cosmetics or toiletries.

Examples of the liquid fatty oils include avocado oil, camellia oil, evening primrose oil, turtle oil, macadamia nut oil, corn oil, mink oil, olive oil, rapeseed oil, egg-yolk oil, sesame oil, wheat germ oil, castor oil, linseed oil, sunflower oil, cottonseed oil, soybean oil, peanut oil, tea seed oil, rice bran oil, jojoba oil, germ oil, triglycerin, glycerin trioctanoate, glycerin triisopalmitate, and the like.

Examples of the solid fats include cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oils, neat's foot oil, Japanese wax, hydrogenated castor oil, and the like.

Examples of the waxes include beeswax, candelilla wax, cotton wax, rice bran wax, carnauba wax, bayberry wax, insect wax, whale wax, lanolin, lanolin acetate, liquid lanolin, lanolin fatty acid isopropyl, hexyl laurate, reduced lanolin, jojoba wax, hard lanolin, POE (polyoxyethylene) lanolin alcohol ether, POE lanolin alcohol acetate, POE cholesterol ether, lanolin fatty acid polyethylene glycol, POE hydrogenated lanolin alcohol ether, and the like.

Examples of the hydrocarbon oils include liquid paraffin, squalene, paraffin, squalane, Vaseline, microcrystalline wax, and the like.

Examples of the higher fatty acids include lauric acid, myristic acid, palmitic acid, stearic acid, oleic acid, 12-hydroxystearic acid, undecylenic acid, tolic acid, isostearic acid, linoleic acid, linolenic acid, eicosapentaenoic acid (EPA), docosahexaenoic acid (DHA), and the like.

Examples of the higher alcohols include linear alcohols such as lauryl alcohol, cetyl alcohol, stearyl alcohol, behenyl alcohol, myristyl alcohol, oleyl alcohol, cetostearyl alcohol, etc.; branched alcohols such as monostearyl glycerin ether, 2-decyltetradecinol, lanolin alcohol, cholesterol, phytosterols, hexyl dodecanol, isostearyl alcohol, octyl dodecanol, etc.; and the like.

Examples of the synthetic ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, di-2-ethylhexyl acid ethylene glycol, dipentaerythritol fatty acid esters, neopentyl glycol dicaprate, diisostearyl malate, glycerin di-2-heptylundecanoate, tri-2-ethylhexyl acid trimethylolpropane, trimethylolpropane triisostearate, tetra-2-ethylhexyl acid pentane erythritol, tri-2-ethylhexyl acid glycerin, trimethylolpropane triisostearate, cetyl 2-ethylhexanoate, 2-ethylhexyl palmitate, glycerin trimyristate, tri-2-heptyl undecanoic acid glyceride, castor oil fatty acid methyl esters, oleic acid oil, cetostearyl alcohol, 2-heptylundecyl palmitate, diisobutyl adipate, N-lauroyl-L-glutamic acid-2-octyldodecyl ester, di-2-heptylundecyl adipate, ethyl laurate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, triethyl citrate, and the like.

Examples of the silicones include linear polysiloxanes such as dimethyl polysiloxane, methyl phenyl polysiloxane, methyl hydrogen polysiloxane, etc.; alicyclic polysiloxanes such as decamethyl polysiloxane, dodecamethyl polysiloxane, tetramethyl tetrahydrogen polysiloxane, etc.; silicone resins and silicone rubbers, in which a three-dimensional network structure is formed; and the like.

Examples of the anionic surfactants include fatty acid soaps such as soap base materials, sodium laurate, sodium palmitate, etc.; higher alkyl sulfate ester salts such as sodium lauryl sulfate, potassium lauryl sulfate, etc.; alkyl ether sulfate ester salts such as triethanolamine POE lauryl sulfate, sodium POE lauryl sulfate, etc.; N-acyl sarcosinates such as sodium lauroyl sarcosinate, etc.; higher fatty acid amide sulfonates such as sodium N-myristoyl-N-methyl taurate, sodium coconut oil fatty acid methyl taurate, sodium lauryl methyl taurate, etc.; phosphate ester salts such as sodium POE oleyl ether phosphate, POE stearyl ether phosphate, etc.; sulfosuccinates such as di-2-ethylhexyl sodium sulfosuccinate, sodium monolauroyl monoethanolamide polyoxyethylene sulfosuccinate, sodium lauryl polypropylene glycol sulfosuccinate, etc.; alkyl benzene sulfonates such as sodium linear dodecylbenzenesulfonate, triethanolamine linear dodecylbenzenesulfonate, linear dodecylbenzenesulfonate, etc.; N-acyl glutamates such as monosodium N-lauroyl glutamate, disodium N-stearoyl glutamate, monosodium N-myristoyl-L-glutamate, etc.; higher fatty acid ester sulfate ester salts such as sodium hydrogenated coconut oil fatty acid glyceryl sulfate, etc.; sulfated oils such as Turkey red oil, etc.; POE alkyl ether carboxylates; POE alkyl allyl ether carboxylates; α-olefin sulfonates; higher fatty acid ester sulfonates; secondary alcohol sulfate ester salts; higher fatty acid alkylolamide sulfate ester salts; sodium lauroyl monoethanolamide succinate; ditriethanolamine N-palmitoyl aspartate; casein sodium; and the like.

Examples of the cationic surfactants include alkyl trimethyl ammonium salts such as stearyl trimethyl ammonium chloride, lauryl trimethyl ammonium chloride, etc.; dialkyl dimethyl ammonium salts such as distearyl dimethyl ammonium chloride, etc.; alkyl pyridinium salts such as poly(N,N'-dimethyl-3,5-methylenepiperidinium)chloride, cetylpyridinium chloride, etc.; alkyl quaternary ammonium salts; alkyl dimethyl benzyl ammonium salts; alkyl isoquinolinium salts; dialkyl morpholinium salts; POE alkylamines; alkylamine salts; polyamine fatty acid derivatives; amyl alcohol fatty acid derivatives; benzalkonium chloride; benzethonium chloride; and the like.

Examples of the amphoteric surfactants include imidazoline-based amphoteric surfactants such as sodium 2-undecyl-N,N,N-(hydroxyethylcarboxymethyl)-2-imidazoline, 2-cocoyl-2-imidazolinium hydroxide-1-carboxyethyloxy-2-sodium salt, etc.; betaine-based amphoteric surfactants such as 2-heptadecyl-N-carboxymethyl-N-hydroxyethylimidazolinium betaine, lauryl dimethyl aminoacetic acid betaine, alkylbetaines, amidobetaine, sulfobetaine, etc.; and the like.

Examples of lipophilic nonionic surfactants include sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, diglycerol sorbitan tetra-2-ethylhexylate, etc.; glycerin polyglycerin fatty acids such as mono-cottonseed fatty acid glycerin, monoerucic acid glycerol, sesquioleic acid glycerol, glyceryl monostearate, α,α'-glyceryl oleate pyroglutamate, glyceryl monostearate malate, etc.; propylene glycol fatty acid esters such as propylene glycol monostearate; hydrogenated castor oil derivatives; glycerin alkyl ethers; and the like.

Examples of hydrophilic nonionic surfactants include POE sorbitan fatty acid esters such as POE sorbitan monooleate, POE sorbitan monostearate, POE sorbitan tetraoleate, etc.; POE sorbitol fatty acid esters such as POE sorbitol monolaurate, POE sorbitol monooleate, POE sorbitol pentaoleate, POE sorbitol monostearate, etc.; POE glycerin fatty acid esters such as POE glycerin monostearate, POE glycerin monoisostearate, POE glycerin triisostearate, etc.; POE fatty acid esters such as POE monooleate, POE distearate, POE monodioleate, ethylene glycol distearate, etc.; POE alkyl ethers such as POE lauryl ether, POE oleyl ether, POE stearyl ether, POE behenyl ether, POE 2-octyldodecyl ether, POE cholestanol ether, etc.; POE alkylphenyl ethers such as POE octylphenyl ether, POE nonylphenyl ether, POE dinonylphenyl ether, etc.; Pluronic surfactants such as Pluronic, etc.; POE/POP alkyl ethers such as POE/POP cetyl ether, POE/POP 2-decyltetradecyl ether, POE/POP monobutyl ether, POE/POP hydrogenated lanolin, POE/POP glycerin ether, etc.; tetra POE/tetra POP ethylenediamine condensates such as Tetronic, etc.; POE castor oil hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamate monoisostearate diester, POE hydrogenated castor oil maleate, etc.; POE beeswax/lanolin derivatives such as POE sorbitol beeswax, etc.; alkanol amides such as coconut oil fatty acid diethanolamide, lauric acid monoethanolamide, fatty acid isopropanolamide, etc.; POE propylene glycol fatty acid esters; POE alkylamines; POE fatty acid amides; sucrose fatty acid esters; POE nonylphenyl formaldehyde condensates; alkyl ethoxy dimethylamine oxides; trioleyl phosphate; and the like.

Examples of the humectants include polyethylene glycol, propylene glycol, glycerin, 1,3-butylene glycol, xylitol, sorbitol, maltitol, chondroitin sulfate, hyaluronic acid, mucoitin sulfuric acid, cholesteryl-12-hydroxystearate, ceramide, glucosylceramide, sodium lactate, bile acid salts, dl-pyrrolidone carboxylates, short-chain soluble collagen, diglycerol (EO) PO adducts, Rosa roxburghii extracts, Achillea millefolium extracts, Melilotus officinalis extracts, and the like.

Examples of natural water-soluble polymer compounds include plant polymer compounds such as gum arabic, gum tragacanth, galactan, carob gum, karaya gum, carrageenan, pectin, agar, quince seed (Cydonia oblonga), algae colloid (brown algae extract), starch (e.g., rice, corn, potato, or wheat), glycyrrhizic acid, etc.; microbial polymer compounds such as xanthan gum, dextran, succinoglucane, pullulan, etc.; and animal polymer compounds such as collagen, casein, albumin, gelatin, etc.

Examples of semi-synthetic water-soluble polymer compounds include starch-based polymer compounds such as carboxymethyl starch, methylhydroxypropyl starch, etc.; cellulose-based polymer compounds such as methyl cellulose, nitrocellulose, ethyl cellulose, methylhydroxypropyl cellulose, hydroxyethyl cellulose, sodium cellulose sulfate, hydroxypropyl cellulose, sodium carboxymethyl cellulose (CMC), crystalline cellulose, cellulose powder, etc.; and alginic acid-based polymer compounds such as sodium alginate, propylene glycol alginate, etc.

Examples of synthetic water-soluble polymer copounds include vinyl-based polymer compounds such as polyvinyl alcohol, polyvinyl methyl ether, polyvinylpyrrolidone, carboxyvinyl polymer (for example, "Carbopol (registered trademark)", manufactured by Lubrizol Advanced Materials, Inc.), etc.; polyoxyethylene-based polymer compounds such as polyethylene glycol 20,000, 4,000,000, or 600,000, etc.; polyoxyethylene-polyoxypropylene copolymers copolymerizing-based polymer compound; acrylic polymer compounds such as sodium polyacrylate, polyethyl acrylate, polyacrylamide, etc.; polyethyleneimine; cationic polymers; and the like.

Examples of inorganic water-soluble polymer compounds include bentonite, AlMg silicate (for example, "Veegum", manufactured by Vanderbilt), laponite, hectorite, silicic anhydride, and the like.

Examples of the thickeners include gum arabic, carrageenan, karaya gum, gum tragacanth, carob gum, pectin, quince seed (Cydonia oblonga), casein, dextrin, gelatin, sodium pectate, sodium alginate, methyl cellulose, ethyl cellulose, hydroxyethyl cellulose, hydroxypropyl cellulose, sodium polyacrylate, carboxyvinyl polymer, dialkyl dimethyl ammonium cellulose sulfate, xanthan gum, magnesium aluminum silicate, bentonite, and the like.

Examples of the ultraviolet absorbers include benzoic acid-based ultraviolet absorbers such as para-aminobenzoic acid (hereinafter abbreviated as "PABA"), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA methyl ester, etc.; anthranilic acid-based ultraviolet absorbers such as homomenthyl-N-acetyl anthranilate, etc.; salicylic acid-based ultraviolet absorbers such as amyl salicylate, menthyl salicylate, homomenthyl salicylate, octyl salicylate, phenyl salicylate, benzyl salicylate, p-isopropanolphenyl salicylate, etc.; cinnamic acid-based ultraviolet absorbers such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, glyceryl mono-2-ethylhexanoyl-di-para-methoxy cinnamate, etc.; benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, 4-hydroxy-3-carboxybenzophenone, etc.; 3-(4'-methylbenzylidene)-d,l-camphor; 3-benzylidene-d,l-camphor; urocanic acid; ethyl urocanate; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 4-methoxy-4'-t-butyldibenzoylmethane; 5-(3,3-dimethyl-2-norbonylidene)-3-pentan-2-one; and the like.

Examples of the ultraviolet screening agents include titanium oxide, talc, carmine, bentonite, kaolin, zinc oxide, and the like.

Examples of the preservatives include methyl paraben, ethyl paraben, and the like.

Examples of the metal ion-sequestering agents include 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and the like.

Examples of the lower alcohols include methanol, ethanol, propanol, isopropanol, isobutyl alcohol, t-butyl alcohol, and the like.

Examples of the polyhydric alcohols include dihydric alcohols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc.; trihydric alcohols such as glycerin, trimethylolpropane, 1,2,6-hexanetriol, etc.; tetrahydric alcohols such as pentaerythritol, etc.; pentahydric alcohols such as xylitol, etc.; hexahydric alcohols such as sorbitol, mannitol, etc.; polyhydric alcohol polymers such as diethylene glycol, dipropylene glycol, triethylene glycol, polypropylene glycol, tetraethylene glycol, diglycerin, polyethylene glycol, triglycerin, tetraglycerin, polyglycerin, etc.; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether, etc.; dihydric alcohol alkyl diethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc.; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc.; glycerin monoalkyl ethers such as chimyl alcohol, selachyl alcohol, batyl alcohol, etc.; sugar alcohols such as sorbitol, maltitol, maltotriose, mannitol, sucrose, erythritol, glucose, fructose, sugars obtained by amylolysis, maltose, xylitose, alcohols obtained by reducing sugars obtained by amylolysis, etc.; glysolid; tetrahydrofurfuryl alcohol; POE tetrahydrofurfuryl alcohol; POP butyl ether; POP/POE butyl ether; tripolyoxypropylene glycerin ether; POP glycerin ether; POP glycerin ether phosphoric acid; POP/POE pentaerythritol ether; and the like.

Examples of monosaccharides include trioses such as D-glyceryl aldehyde, dihydroxyacetone, etc.; tetroses such as D-erythrose, D-erythrulose, D-threose, erythritol, etc.; pentoses such as L-arabinose, D-xylose, L-lyxose, D-arabinose, D-ribose, D-ribulose, D-xylulose, L-xylulose, etc.; hexoses such as D-glucose, D-talose, D-psicose, D-galactose, D-fructose, L-galactose, L-mannose, D-tagatose, etc.; heptoses such as aldoheptose, heptulose, etc.; octoses such as octulose, etc.; deoxy sugars such as 2-deoxy-D-ribose, 6-deoxy-L-galactose, 6-deoxy-L-mannose, etc.; amino sugars such as D-glucosamine, D-galactosamine, sialic acid, aminouronic acid, muramic acid, etc.; uronic acids such as D-glucuronic acid, D-mannuronic acid, L-guluronic acid, D-galacturonic acid, L-iduronic acid, etc.; and the like.

Examples of oligosaccharides include sucrose, gentianose, umbelliferose, lactose, planteose, isolychnoses, α,α-trehalose, raffinose, lychnoses, umbilicin, stachyose, verbascoses, and the like.

Examples of polysaccharides include cellulose, quince seed, chondroitin sulfate, starch, galactan, dermatan sulfate, glycogen, gum arabic, heparan sulfate, hyaluronic acid, tragacanth gum, chondroitin, xanthan gum, mucoitin sulfuric acid, guar gum, dextran, charonic acid, and the like.

Examples of amino acids include neutral amino acids such as glycine, alanine, valine, leucine, isoleucine, serine, threonine, tryptophan, cystine, cysteine, methionine, proline, hydroxyproline, etc.; acidic amino acids such as aspartic acid, glutamic acid, asparagine, glutamine, etc.; and basic amino acids such as arginine, histidine, lysine, hydroxylysine, etc.

Examples of the amino acid derivatives include sodium acylsarcosine (sodium lauroylsarcosine), acylglutamic acid salts, sodium acyl-β-alanine, glutathione, pyrrolidonecarboxylic acid, and the like.

Examples of the organic amines include monoethanolamine, diethanolamine, triethanolamine, morpholine, triisopropanolamine, 2-amino-2-methyl-1,3-propanediol, 2-amino-2-methyl-1-propanol, and the like.

Examples of the synthetic resin emulsions include an acrylic resin emulsion, a polyethyl acrylate emulsion, an acrylic resin liquid, a polyacrylalkyl ester emulsion, a polyvinyl acetate resin emulsion, and the like.

Examples of the pH adjusters include buffers such as lactic acid/sodium lactate, citric acid/sodium citrate, etc.; and the like.

Examples of the vitamins include vitamin A such as vitamin A oil, retinol, etc.; vitamin B1 such as thiamine, etc.; vitamin B2 such as riboflavin, etc.; vitamin B6 such as pyridoxine hydrochloride, etc.; vitamin C such as L-ascorbic acid, L-ascorbic acid phosphate ester, L-ascorbic acid monopalmitate, L-ascorbic acid dipalmitate, L-ascorbic acid-2-glucoside, etc.; pantothenic acids such as calcium pantothenate, etc.; vitamin D such as vitamin D2, cholecalciferol, etc.; vitamin E such as α-tocopherol, tocopheryl acetate, and DL-α-tocopheryl nicotinate, etc.; pantothenic acid and derivatives thereof; biotin; and the like.

Examples of the antioxidants include tocopherols, dibutylhydroxytoluene, butylhydroxyanisole, gallic acid esters, and the like.

Examples of the antioxidant aids include phosphoric acid, citric acid, ascorbic acid, maleic acid, malonic acid, succinic acid, fumaric acid, cephalin, hexametaphosphate, phytic acid, ethylenediaminetetraacetic acid, and the like.

The fragrance or cosmetic, the skin external agent, and so on in the use of the present invention are arbitrary in terms of a form and can be made in various forms such as a solution system, a soluble form system, an emulsion system, an oil liquid system, a gel system, a powder dispersion system, a water-oil two-layer system, a water-oil-powder three-layer system, etc., by blending the essential component with one or more of the above-described arbitrary blending components depending on the intended purpose by an ordinary method.

A fragrance composition can be prepared from the melanin production inhibitor by mixing with one or more of commonly used fragrance components.

Examples of the "commonly used fragrance components" as referred to herein include various synthetic fragrances, naturally occurring essential oils, synthetic essential oils, citrus oils, animal fragrances, and the like. For example, it is possible to use a wide variety of fragrance components such as those described in "Perfume and Flavor Chemicals (Aroma Chemicals) 1, 2" (Steffen Arctender (1969)), "GOUSEI KOURYOU, KAGAKU TO SHOUHIN CHISHIKI (Synthetic Flavors and Fragrances, Chemistry and Product Knowledge) <Enlarged and Revised Edition>" (authored by Indo Motoichi, published as an enlarged and revised edition on March 22, 2005 by The Chemical Daily Co., Ltd.), or "Collection of Well-known Prior Arts (Flavors and Fragrances) Part I" (published on Jan. 29, 1999, by Japan Patent Office). Of these fragrance components, typical examples include α-pinene, limonene, cis-3-hexenol, phenylethyl alcohol, styralyl acetate, ammonium isovalerate, eugenol, rose oxide, linalool, benzaldehyde, muscone, Musk T (registered trademark of Takasago International Corporation), and Thesaron (registered trademark of Takasago International Corporation), as well as fragrances having cooling sensation effect such as menthol and derivatives having a menthane skeleton, etc. The use of such a fragrance component in combination with the melanin production inhibitor makes it possible to improve the odor quality and note of the prepared fragrance.

In addition, multiple steps including tyrosinase-related steps, transfer to the epidermis, and the like are involved in the skin-whitening effect. Accordingly, it is possible to enhance the skin-whitening activity by using a mixture of multiple skin-whitening agents selected from various skin-whitening agents including the representative compounds having skin-whitening activity described in the section of Background Art.

### Examples

The present invention is hereunder specifically described by way of Examples, but it should be construed that the present invention is by no means limited by these Examples. In addition, the present invention may be altered or modified in many ways without deviating from the scope of the present invention. With regard to the units in the formulations described below, the term "%" means "% by mass", and a composition ratio indicates a mass ratio, unless specifically mentioned.

### <Test Example 1> Evaluation for Melanin Production-Inhibitory Activity against Pigment Cells

With respect to the components (compounds) of Examples 1 to 3 and Reference Example 1 shown in Table 1, the following test was performed, thereby studying the evaluation for melanin production-inhibitory activity.

In a plastic culture flask (25 cm²), B-16 melanoma cells in a number of 1 × 10⁵ were seeded and cultured in a 10% serum-containing DMEM medium (manufactured by Nippon Suisan Kaisha, Ltd.) in the presence of 5% carbon dioxide at 37°C. One day after the culture, an evaluation sample diluted with ethanol was added, and the cells were further cultured for 4 days. After completion of the culture, the medium was removed, and the cells were rinsed with PBS (phosphate buffer solution). Thereafter, the cells were detached from the plate by using a medium containing trypsin and EDTA (ethylenediaminetetraacetic acid) and harvested by means of centrifugation. The resulting cells were rinsed three times with 1 mL of 5% trichloroacetic acid controlled at 4°C, rinsed twice with 1 mL of an ethanol/diethyl ether (3/1) solution, and then rinsed once with 1 mL of diethyl ether. After drying, 1 mL of 2N sodium hydroxide was added to dissolve the cells at 60°C. After allowing the solution to stand for cooling, an absorbance at 415 nm was measured, thereby measuring the amount of melanin. Each measured value was calculated as a relative value (%) relative to a value of a negative control in which the evaluation sample was not exposed. The evaluation was performed by three times repetition. The evaluation results are shown in Table 1.

**Table 1**

| | | Component (compound) | Concentration (µg/mL) | Melanin inhibition ratio (%) |
|---|---|---|---|---|
| Example | 1 (reference) | Vetiver oil | 3.13 | 53 |
| | | | 6.25 | 84 |
| | 2 | Cabreuva oil | 1.56 | 24 |
| | | | 3.13 | 50 |
| | 3 (reference) | Cedarwood Virginia oil | 6.25 | 52 |
| | | | 12.5 | 81 |
| Reference Example | 1 | Arbutin | 3.13 | 39 |
| | | | 6.25 | 50 |
| | | | 12.5 | 71 |

As is clear from Table 1, the vetiver oil, the cabreuva oil, and the cedarwood Virginia oil exhibited an excellent skin-whitening effect equal to or higher than arbutin.

### <Test Example 2> Evaluation for Skin-Whitening Activity Using Melanocyte-Containing Human Three-dimensional Cultured Epidermal Tissue

A melanocyte-containing human three-dimensional cultured epidermal model, LabCyte MELANO-MODEL 24 (manufactured by Japan Tissue Engineering Co., Ltd.) was cultured in accordance with an instruction manual. That is, a culture cup having a human cultured epidermis charged therein was soaked in 1.5 mL of an improved melanin production-promoting medium, and an evaluation sample was added in the cup and exposed, followed by culture at 37°C for 2 weeks in the presence of 5% carbon dioxide. The medium and the evaluation sample were exchanged once every two to three days. After completion of the culture, the number of living cells and the amount of melanin were measured according to the MTT assay by a method described in the instruction manual. Each measured value was calculated as a relative value (%) relative to a value of a negative control in which the evaluation sample was not exposed. The evaluation was performed by three times repetition. The evaluation results are shown in Table 2.

**Table 2**

| Evaluation sample | Number of living cells (%) | Amount of melanin (%) |
|---|---|---|
| Vetiver oil, 0.125% (reference) | 94.6 ± 6.8 | 40.5 ± 0.8 |
| Cabreuva oil, 0.125% | 96.6 ± 14.9 | 57.2 ± 5.5 |
| Cedarwood Virginia oil, 0.25% (reference) | 105.2 ± 3.5 | 45.2 ± 4.0 |
| Kojic acid, 0.10% (positive control) | 92.3 ± 8.8 | 48.4 ± 4.0 |

As is clear from the results shown in Table 2, the vetiver oil, the cabreuva oil, and the cedarwood Virginia oil inhibited the melanin production at a concentration free from influences of cytotoxicity.

### <Test Example 3> Evaluation Method of Tyrosinase Activity Inhibiting Action

In a plastic culture flask (25 cm²), B-16 melanoma cells in a number of 1 × 10⁵ were seeded and cultured in the presence of 5% carbon dioxide at 37°C for 5 days. Thereafter, the culture medium was removed, and the resultant was rinsed with 3 mL of PBS. Thereafter, the cells were detached with trypsin and EDTA and harvested by means of centrifugation. The resulting cells were regulated with an assay buffer containing 0.5% Triton X-100 (0.1 M sodium phosphate aqueous solution) cooled at 4°C, such that the number of cells was 6 × 10⁵ per mL and dissolved to prepare a crude enzyme solution. 10 µL of an evaluation sample having been regulated to 0.2% with ethanol was dispersed in each well of a 96-well plate, and 40 µL of the assay buffer, 75 µL of the crude enzyme solution, and 75 µl of an assay buffer containing 0.25% L-DOPA were added. An absorbance at 415 nm was measured immediately after the addition and after a reaction at 37°C for 6 hours, respectively, thereby quantitating the amount of melanin. Each measured value was calculated as a relative value (%) relative to a value of a negative control in which the evaluation sample was not exposed. The evaluation was performed by three times repetition. The evaluation results are shown in Table 3.

**Table 3**

| Evaluation sample (final concentration: 100 µg/mL) | Tyrosinase activity inhibition ratio (%) |
|---|---|
| Vetiver oil (reference) | 111 + 7.6 |
| Cabreuva oil | 98 ± 7.8 |
| Cedarwood Virginia oil (reference) | 111 ± 7.7 |

As is clear from the results shown in Table 3, it was indicated that the vetiver oil, the cabreuva oil, and the cedarwood Virginia oil do not inhibit the tyrosinase activity.

### <Test Example 4> Evaluation Method of Tyrosinase Biosynthesis Inhibiting Action

In a 24-well plate, B-16 melanoma cells in a number of 2 × 10⁴ were seeded and cultured in the presence of 5% carbon dioxide at 37°C for one day. Thereafter, medium exchange with 800 µL of a culture medium containing an evaluation sample was performed, and the cells were cultured under the same conditions for 2 days. The culture solution was removed and rinsed twice with 1 mL of PBS. Thereafter, 150 µL of an assay buffer containing 0.5% Triton X-100 cooled at 4°C was added to dissolve the cells, and a supernatant resulting from centrifugation was prepared as a cell extract. A protein mass of the cell extract was quantitated by using Pierce BCA Protein Assay Kit (manufactured by Thermo Fisher Scientific Inc.), 100 µl of the cell extract was then dispersed in each well of a 96-well plate, and 100 µl of an assay buffer containing 0.5% L-DOPA was added. An absorbance at 415 nm was measured immediately after the addition and after a reaction at 37°C for 3 hours, respectively, thereby quantitating the amount of melanin. The tyrosinase biosynthesis amount in each evaluation sample addition group was determined as the melanin production amount per unit protein and further calculated as a relative value (%) relative to a value of a negative control in which the evaluation sample was not exposed. The evaluation was performed by three times repetition. The evaluation results are shown in Table 4.

**Table 4**

| Evaluation sample | Concentration (µg/mL) | | | |
|---|---|---|---|---|
| | 1.56 | 3.13 | 6.25 | 12.5 |
| Vetiver oil (reference) | - | 136 ± 3.0% | 151 ± 2.8% | - |
| Cabreuva oil | 117 ± 3.9% | 119 ± 10.2% | - | - |
| Cedarwood Virginia oil (reference) | - | - | 108 ± 2.1% | 105 ± 3.2% |

As is clear from Table 4, it was indicated that the vetiver oil, the cabreuva oil, and the cedarwood Virginia oil do not inhibit the tyrosinase biosynthesis.

### <Formulation Reference Example 1> Skin-Whitening Lotion

A skin-whitening lotion was prepared by dissolving the components shown in the following Table 5 at room temperature with stirring. In Table 5, the vetiver oil of Reference Example 1 was used as the melanin production inhibitor.

**Table 5**

| | Component | Blending amount (% by mass) |
|---|---|---|
| 1 | Glycerin | 10.0 |
| 2 | 1,3-Butylene glycol | 5.0 |
| 3 | Glucose | 2.0 |
| 4 | Ethanol | 5.0 |
| 5 | Carboxyvinyl polymer | 0.02 |
| 6 | Dipotassium glycyrrhizate | 0.1 |
| 7 | Sodium hyaluronate | 0.001 |
| 8 | Melanin production inhibitor | 0.1 |
| 9 | Citric acid | 0.05 |
| 10 | Sodium citrate | 0.1 |
| 11 | Potassium hydroxide | 0.01 |
| 12 | Purified water | Balance |

### <Formulation Reference Example 2> Skin-Whitening Cream

A skin-whitening cream was prepared by dissolving the components shown in the following Table 6 at room temperature with stirring. In Table 6, the vetiver oil of Reference Example 1 was used as the melanin production inhibitor.

**Table 6**

| | Component | Blending amount (% bv mass) |
|---|---|---|
| 1 | Stearyl alcohol | 6.0 |
| 2 | Stearic acid | 2.0 |
| 3 | Squalene | 10.0 |
| 4 | Octyl dodecanol | 5.0 |
| 5 | Olive oil | 5.0 |
| 6 | 1,3-Butylene glycol | 8.0 |
| 7 | Polyethylene glycol 1500 | 4.0 |
| 8 | POE (25) cetyl alcohol ether | 3.0 |
| 9 | Glyceryl monostearate | 3.0 |
| 10 | Melanin production inhibitor | 0.1 |
| 11 | Purified water | Balance |

### <Formulation Reference Example 3> Skin-Whitening Pack

A skin-whitening pack was prepared by dissolving the components shown in the following Table 7 at room temperature with stirring. In Table 7, the vetiver oil of Reference Example 1 was used as the melanin production inhibitor.

**Table 7**

| | Component | Blending amount (% bv mass) |
|---|---|---|
| 1 | Polyvinyl alcohol | 15.0 |
| 2 | Carboxymethyl cellulose | 5.0 |
| 3 | 1,3-Butylene glycol | 5.0 |
| 4 | Ethanol | 12.0 |
| 5 | Melanin production inhibitor | 0.05 |
| 6 | POE oleyl alcohol ether | 0.5 |
| 7 | Citric acid | 0.02 |
| 8 | Sodium citrate | 0.04 |
| 9 | Purified water | Balance |

### <Formulation Example 4> Skin-Whitening Lotion

A skin-whitening lotion was prepared by dissolving the components shown in the following Table 8 at room temperature with stirring. In Table 8, the cabreuva oil of Example 2 was used as the melanin production inhibitor.

**Table 8**

| | Component | Blending amount (% by mass) |
|---|---|---|
| 1 | Quince seed extract | 8.0 |
| 2 | Glycerin | 3.0 |
| 3 | 1,3-Butylene glycol | 5.0 |
| 4 | Melanin production inhibitor | 0.15 |
| 5 | Polyoxyethylene sorbitan lauric acid ester | 1.2 |
| 6 | Ethanol | 5.0 |
| 7 | Methyl paraben | 0.2 |
| 8 | Citrus fragrance | 0.1 |
| 9 | Purified water | Balance |

### <Formulation Example 5> Skin-Whitening Milky Lotion

A skin-whitening milky lotion was prepared by dissolving the components shown in the following Table 9 at room temperature with stirring. In Table 9, the cabreuva oil of Example 2 was used as the melanin production inhibitor.

**Table 9**

| | Component | Blending amount (% by mass) |
|---|---|---|
| 1 | Castor oil | 1.0 |
| 2 | Squalene | 2.0 |
| 3 | Behenyl alcohol | 1.0 |
| 4 | Caprylic/capric triglyceride | 2.0 |
| 5 | Tetraglycerin-condensed ricinolate | 0.1 |
| 6 | Propylene glycol monooleate | 0.5 |
| 7 | Glyceryl monostearate | 1.0 |
| 8 | Hexaglyceryl monomyristate | 1.0 |
| 9 | Decaglyceryl monomyristate | 0.5 |
| 10 | Propyl paraoxybenzoate | 0.1 |
| 11 | Quince seed extract | 5.0 |
| 12 | Melanin production inhibitor | 0.03 |
| 13 | 1,3-Butylene glycol | 3.0 |
| 14 | Methyl paraoxybenzoate | 0.1 |
| 15 | Rose fragrance | 0.1 |
| 16 | Purified water | Balance |

### <Formulation Example 6> Bath Additive

A bath additive was prepared by dissolving the components shown in the following Table 10 at room temperature with stirring. In Table 10, the cabreuva oil of Example 2 was used as the melanin production inhibitor.

**Table 10**

| | Component | Blending amount (% bv mass) |
|---|---|---|
| 1 | Dry sodium sulfate | 40.0 |
| 2 | Sodium hydrogen carbonate | 57.5 |
| 3 | Olive oil | 0.2 |
| 4 | Melanin production inhibitor | 0.1 |
| 5 | Light silicic anhydride | 0.3 |
| 6 | Floral fragrance | 1.7 |
| 7 | Yellow No. 202 (1) | 0.2 |

### <Formulation Example 7> Cream

A cream fragrance composition having a high-grade image of a floral green type of a component composition shown in the following Table 11 was prepared by an ordinary method. In Table 11, the cabreuva oil of Example 2 was used as the melanin production inhibitor.

**Table 11**

| | Component | Blending amount (% by mass) |
|---|---|---|
| 1 | Hydroxycitronellal | 3.0 |
| 2 | Linalool | 3.0 |
| 3 | Linalyl acetate | 4.0 |
| 4 | Nerolidol | 4.0 |
| 5 | Rose Fragrance Base (manufactured by Takasago International Corporation) | 14.5 |
| 6 | Tonalid (manufactured by PFW) | 5.0 |
| 7 | Preparation Base (manufactured by Takasago International Corporation) | 9.5 |
| 8 | Benzyl salicylate | 7.0 |
| 9 | Galaxolide (manufactured by IFF) | 4.0 |
| 10 | Methyl dihydrojasmonate | 5.0 |
| 11 | Heliofresh (manufactured by Ube Industries, Ltd.) | 15.0 |
| 12 | Dihydromyrcenol | 3.0 |
| 13 | Dipropylene glycol | 10.0 |
| 14 | Farnesol | 1.75 |
| 15 | Cis-3-hexenyl salicylate | 4.5 |
| 16 | Lilial (manufactured by Givaudan) | 5.5 |
| 17 | Melanin production inhibitor | 1.25 |

Next, a cream having a component composition shown in the following Table 12 was prepared by using the cream fragrance composition prepared as described above by an ordinary method.

**Table 12**

| | Component | Blending amount (% by mass) |
|---|---|---|
| 1 | Stearic acid | 1.0 |
| 2 | Cholesteryl isostearate | 2.0 |
| 3 | Jojoba oil | 4.0 |
| 4 | Squalene | 8.0 |
| 5 | Sorbitan sesquioleate | 0.8 |
| 6 | Polyoxyethylene sorbitan monostearate (20 E.O.) | 1.2 |
| 7 | 1,3-Butylene glycol | 5.0 |
| 8 | Methyl paraben | 0.25 |
| 9 | L-Arginine | 0.4 |
| 10 | Carboxyvinyl polymer | 0.2 |
| 11 | Cream fragrance composition | 0.1 |
| 12 | Purified water | Balance |

## Claims

1. External use of one active ingredient which is cabreuva oil for inhibiting melanin production in skin.

2. The use according to claim 1, in which said active ingredient is used in a proportion of 10 % by mass or more.

3. The use according to claim 1 or 2, in which said active ingredient is mixed with other conventional melanin production inhibitor selected from the group consisting of pantetheine-s-sulfonic acid, isoferulic acid, ascorbic acid, hydroquinone, arbutin, kojic acid, linoleic acid (ester), ellagic acid, glycyrrhizic acid, lactic acid (ester), 4-alkylresorcinols, licorice extract and placenta extract.

4. The use according to any one of claims 1 to 3, in which the active ingredient is blended with bases to produce a product selected from the group consisting of a fragrance composition, a cosmetic, a skin-whitening agent, a skin external agent, skin-whitening emulsion cosmetics, skin-whitening creams, skin-whitening lotions, skin-whitening oil-based cosmetics, skin-whitening packs, skin-whitening foundations, or blended with other components including powder components, liquid fatty oils, solid fats, waxes, hydrocarbon oils, higher fatty acids, higher alcohols, synthetic ester oils, silicones, anionic surfactants, cationic surfactants, amphoteric surfactants, nonionic surfactants, humectants, water-soluble polymer compounds, thickeners, coating agents, ultraviolet absorbers, ultraviolet screening agents, preservatives, metal ion-sequestering agents, lower alcohols, polyhydric alcohols, saccharides, amino acids, organic amines, synthetic resin emulsions, pH adjusters, skin nutrients, vitamins, antioxidants, antioxidant aids, fragrances, water.

## Patentansprüche

1. Externe Verwendung eines aktiven Wirkstoffs, des Cabreuva Öls, zur Hemmung der Melaninproduktion in der Haut.

2. Verwendung nach Anspruch 1, bei der der aktive Wirkstoff in einem Anteil von 10 Masse-% oder mehr verwendet wird.

3. Verwendung nach Anspruch 1 oder 2, wobei der aktive Wirkstoff mit einem anderen herkömmlichen Inhibitor der Melaninproduktion gemischt wird, der aus der Gruppe ausgewählt ist, die aus Pantetheinsulfonsäure, Isoferulasäure, Ascorbinsäure, Hydrochinon, Arbutin, Kojisäure, Linolsäure (Ester), Ellagsäure, Glycyrrhizinsäure, Milchsäure (Ester), 4-Alkylresorcinols, Süßholzextrakt und Plazentaextrakt besteht.

4. Verwendung nach einem der Ansprüche 1 bis 3, bei der der aktive Wirkstoff mit Basen gemischt wird, um ein Produkt herzustellen, das aus der Gruppe ausgewählt ist, bestehend aus einer Duftstoffzusammensetzung, einem Kosmetikum, einem Mittel zur Hautaufhellung, einem Mittel zur äußerlichen Anwendung auf der Haut, hautaufhellenden Emulsionskosmetika, hautaufhellenden Cremes, hautaufhellenden Lotionen, hautaufhellenden Kosmetika auf Ölbasis, hautaufhellenden Packungen, hautaufhellenden Foundations, oder mit anderen Komponenten einschließlich Pulverkomponenten einbezogen, flüssige fette Öle, feste Fette, Wachse, Kohlenwasserstofföle, höhere Fettsäuren, höhere Alkohole, synthetische Esteröle, Silikone, anionische Tenside, kationische Tenside, amphotere Tenside, nichtionische Tenside, Feuchthaltemittel, wasserlöslichen Polymerverbindungen, Verdickungsmitteln, Beschichtungsmitteln, Ultraviolettabsorbern, Ultraviolettabschirmmitteln, Konservierungsmitteln, Metallionensequestriermitteln, niederen Alkoholen, mehrwertigen Alkoholen, Sacchariden, Aminosäuren, organischen Aminen, Kunstharzemulsionen, pH-Einstellern, Hautnährstoffen, Vitaminen, Antioxidantien, Antioxidationsmitteln, Duftstoffen, Wasser.

## Revendications

1. Utilisation externe d'un ingrédient actif qui est de l'huile de cabreuva pour inhiber la production de mélanine dans la peau.

2. Utilisation selon la revendication 1, dans laquelle ledit ingrédient actif est utilisé selon une proportion de 10 % en masse ou plus.

3. Utilisation selon la revendication 1 ou 2, dans laquelle ledit ingrédient actif est mélangé avec un autre inhibiteur de production de mélanine classique choisi dans le groupe constitué d'acide pantéthéine-s-sulfonique, d'acide iso-férulique, d'acide ascorbique, d'hydroquinone, d'arbutine, d'acide kojique, d'acide linoléique (ester), d'acide ellagique, d'acide glycyrrhizique, d'acide lactique (ester), de 4-alkylrésorcinols, d'extrait de réglisse et d'extrait de placenta.

4. Utilisation selon l'une quelconque des revendications 1 à 3, dans laquelle l'ingrédient actif est mélangé avec des bases pour produire un produit choisi dans le groupe constitué d'une composition parfumée, d'un cosmétique, d'un agent blanchissant la peau, d'un agent externe pour la peau, d'émulsions cosmétiques blanchissant la peau, de crèmes blanchissant la peau, de lotions blanchissant la peau, de cosmétiques à base d'huile blanchissant la peau, de compresses blanchissant la peau, de fonds de teint blanchissant la peau, ou mélangé avec d'autres composants incluant des composants en poudre, des huiles grasses liquides, des graisses solides, des cires, des huiles hydrocarbonées, des acides gras supérieurs, des alcools supérieurs, des huiles d'ester synthétique, des silicones, des tensioactifs anioniques, des tensioactifs cationiques, des tensioactifs amphotères, des tensioactifs non-ioniques, des humectants, des composés polymères hydrosolubles, des épaississants, des agents d'enrobage, des absorbeurs d'ultraviolets, des agents de filtrage des ultraviolets, des conservateurs, des agents séquestrant des ions métalliques, des alcools inférieurs, des alcools polyhydriques, des saccharides, des acides aminés, des amines organiques, des émulsions de résine synthétique, des ajusteurs de pH, des nutriments pour la peau, des vitamines, des antioxydants, des aides antioxydantes, des parfums, de l'eau.
